# EUROPEAN PATENT APPLICATION

(11) **EP 4 008 386 A1**
(43) Date of publication of application: **08.06.2022**
(21) Application number: 21150439.4
(22) Date of filing: 06.01.2021
(51) Int. Cl.: A61M 21/02, A61N 5/06

(54) **DEVICE FOR PROMPT RECOVERY FROM SURGICAL OPERATIONS UNDER TOTAL OR DEEP ANESTHESIA OR FOR GENERAL INDIVIDUAL RELAXATION**

(30) Priority: 07.12.2020 IT 202000030077
(71) Applicant: The Eyes Republic, la repubblica degli occhi S.r.l.s., 33170 Pordenone (IT)
(72) Inventor: Perazza, Roberto, 33170 Pordenone (IT); Vanin, Stefano, 33170 Pordenone (IT)
(74) Representative: Leganza, Alessandro

(57) **Abstract**

Device (1) for relaxation and/or for surgical operations under deep anesthesia and/or for the treatment of depression and/or for dysrhythmia and/or for the treatment of jet lag disorders, comprises:
- a frame (2) suitable for covering the part of the face where the eyeballs are located, and
- two distinct light emission sources (3, 4) arranged on the internal surface of the frame and in front of the eyeballs suitable for emitting light,
wherein the light sources each have a surface area comprised from 0.15mm² to 1cm², or from 0.5mm² to 5mm², or from 0.5mm² to 2mm² .

## Description

### Technical field

The present invention is placed in the field of devices, in particular of devices designed to improve some conditions of people, such as relaxation, post-operative recovery, depression, dysrhythmia or the Jet Lag effect.

### Backgroud of the invention

Nowadays, when a patient has to undergo a major surgery, he/she is treated with total anesthesia, which involves awaking of the patient after a certain number of hours and said awakening is very often accompanied by a situation of dizziness and disorientation.

Indeed, especially in patients subject to deep sedation, they take many hours to resume normal post-surgery and post-anesthesia functions.

Many individuals are subject to depression, and in order to treat or alleviate the symptoms caused by it, they take anxiolytic substances or sleeping pills, often belonging to the benzodiazepine class, as Diazepam, Lorazepam, Medazepam, etc.

However, said substances, also classified in table IV of narcotic substances, also cause other disorders, the first of which is a form of drug dependence.

Finally, nowadays, many individuals who, especially for work reasons, are forced to travel often, are subject to discomfort due to dysrhythmia or jet lag, i.e. a condition of imbalance in the time zone, and therefore a phase shift in the circadian rhythm of the sleep. These disorders are often treated or alleviated with drugs including Melatonin.

Nowadays, all the different situations above described require the adoption of different various devices and/or drugs, as for example various tablets of different drugs, rather than the assistance by spouses or medical staff for people who wake up after deep sedation.

Finally, it would be optimal to have a device which, in addition to solving or at least alleviating all three situations above described, at the same time, also allows to improve relaxation by the subjects who have said device.

### Summary of the invention

The problem addressed by the present invention is therefore that of providing a device which allows to solve all the aforementioned problems at the same time, or rather which allows to solve or effectively improve all the situations listed above, and moreover, which allows to obtain improvement in relaxation of individuals.

Therefore, the present invention solves the aforementioned problem by means of the device (1) and the methods that see it used as outlined in the annexed claims, the definitions of which are an integral part of the present description, thus allowing to provide a single device in order to improve the post-operative recovery, depression, dysrhythmia and/or jet lag and finally, to allow relaxation of individuals.

The finding of the present invention, that is the device (1), show indeed extremely practical, small, light and easily transportable.

This invention, using already commercially available materials, therefore will be particularly advantageous in terms of economy for large-scale applications industrial.

In particular, the invention relates to a device (1) for relaxation and/or for the post-operative recovery from surgery under deep anesthesia and/or for the treatment of depressions and/or dysrhythmia and/or for the treatment of jet lag disorders, comprising:
- a frame (2) suitable for covering the part of the face where the eyeballs are located,
- two distinct light emission sources (3, 4) arranged on the internal surface of the frame and in front of the eyeballs suitable for emitting light,
wherein the light sources (3, 4) each have a surface area comprised from 0.15 mm² to 1 cm², or from 0.5 mm² to 5 mm², or from 0.5 mm² to 2 mm².

Another object is a method for manufacturing the device (1) comprising or, alternatively, consisting of the following steps:
A) providing a first layer of a frame (2),
B) applying on said first layer of a frame (2), a microprocessor (5), a battery (6), tracks suitable for allowing the passage of electric current (7),
C) covering the layer obtained in step B) with a second layer of material.

Another object is a method for relaxation comprising or, alternatively, consisting of the following steps:
a) wearing a device (1) by an individual;
b) operating the device (1) so that it emits intermittent blue light.
c) keeping the intermittent blue light emission for at least 10 minutes.

Finally, another object is the use of the device (1) to facilitate the post-operative recovery of surgical interventions under deep anesthesia or for relaxation of individuals.

Additional features and advantages of the device (1) of the invention and of the methods use will result from the description of the embodiment examples of the invention, provided as an indication of the invention.

### Brief description of the figures

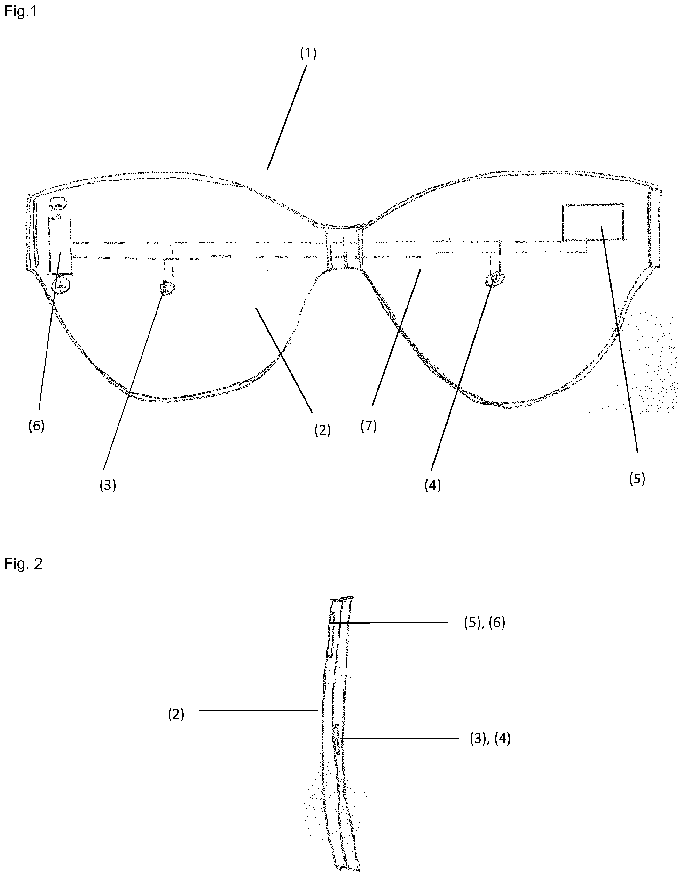

Fig.1 shows a front perspective view of the device (1) comprising:
   - a frame (2) for glasses with a standard external shape, entirely solid and with a defined thickness. Said frame (2) is designed to place a pair of temples to fit the device and/or an elastic band to wrap the skull without contact between the device and the eyelids or eyeballs but a wearable visor consists of:
   - two light sources (3, 4) arranged on the internal surface of the frame (2),
   - a microprocessor or microchip (5) arranged inside the frame (2),
   - a battery (6) arranged inside the frame (2),
   - tracks suitable for allowing the passage of the electric current (7), arranged inside the frame (2).
Fig. 2 shows a perspective view of the side of the device (1) thus comprising a frame (2), wherein two layers of the frame (2) are highlighted, wherein the microprocessor (5) and battery (6) are located on the inner side of the outer layer, while the two light sources (3, 4) are located inside or on the inner side of the inner layer.

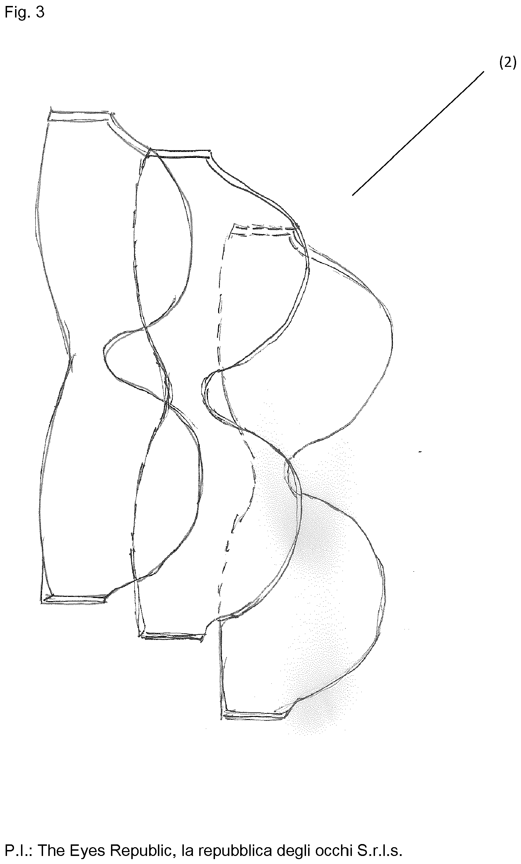

Fig.3 shows an exploded view of the device (1) wherein the frame (2) comprises 3 distinct layers of materials, which once assembled, provide a multilayer frame (2) comprising 3 layers of superimposed material.

### Description of the invention

For the purposes of the present document, the terms "upper", "lower", "right", "left", "rear", "front", "internal", "external", "vertical", "horizontal" and its derivatives refer to the oriented concepts in Fig. 1.

However, it should be understood that concepts can take various alternative orientations, unless where expressly noted otherwise.

As used in this document, the term "and/or", when used in a list of two or more items, means that any of the listed items can be used alone, or in any combination of two or more of the listed items.

For example, whether a composition is described as containing components A, B and/or C, or, A, and/or B and/or C, the composition may contain only A; only B; only C; A and B in combination; A and C in combination; B and C in combination; or A, B and C in combination.

The terms "comprises", "comprising" or any other variation thereof, means to cover a non-exclusive inclusion, so that a device, method, use, etc. which includes a list of elements, that does not only include those elements, but may include other elements not expressly listed or related to that device, method, use, etc.

An element followed by "comprises ... a ..." does not prevent, without further constraints, the existence of further identical elements in the device, method or use which comprises the element.

An object of the present invention is a device (1) for relaxation and/or for post-operative recovery under deep anesthesia and/or for the treatment of depression and/or dysrhythmia and/or for the treatment of jet lag disorders, comprising:
- a frame (2) suitable for covering the part of the face where the eyeballs are located,
- two distinct light emission sources (3, 4) arranged on the internal surface of the frame and in front of the eyeballs suitable for emitting light,
wherein the light sources (3, 4) each have a surface area comprised from 0.15 mm² to 1 cm², or from 0.5 mm² to 5 mm², or from 0.5 mm² to 2 mm².

Indeed, it has been surprisingly found that the device (1) allows to satisfy all of the following situations individually or simultaneously:
- allowing a much faster post-operative recovery,
- mitigating the effects of depression,
- mitigating the effects of dysrhythmia,
- mitigating the effects of jet lag disorders,
and moreover, improving general relaxation for individuals,
with a single device, thus avoiding to have to use different devices and/or drugs to treat different situations.

In particular, it has been found that the device (1) of the present invention allows:
- a much faster post-operative recovery for all those subjects who have undergone surgery in total sedation,
- to mitigate the effects of depression for all subjects suffering from depression;
- to mitigate the effects of jet lag disorders for all intercontinental travellers subject to jet lag disorders;
and moreover, to improve relaxation for individuals,

Indeed, in the case of total sedation, the device (1), worn at the beginning of the surgical intervention and kept on for the entire duration of the surgery, it allows the reduction of post-operative recovery times from 40% to 70% lower than average times found for the return to normal cognitive activity.

The age of the subjects significantly affects recovery times, subjects over 60 years old will benefit more from using the device (1).

Young subjects show sensitively noticeable results and benefits, although there are not striking as those of the over 60/70/80/90 years old.

In particular, the blue intermittent light device (1) used during operations on patients in deep sedation decreases post-operative recovery times by limiting those disorders due to the deep sedation, i.e. the phase shift of the biological clock.

The phase shift of the biological clock is more marked in subjects over 50 and the age is older, greater are the recovery disorders.

For subjects suffering from depression, instead, the operating modes are different, the subject suffering from depression will have to use the device (1) upon awakening, 10/15 minutes of preventive treatment to realign the circadian clock and relieve those symptoms of chronic fatigue typical of the subject suffering from depression.

The device (1), with the same light frequencies, allows subjects suffering from depression to recover concentration and attention in less time. The difficulty of depressed subjects is at the awakening, since they have extended times of biological realignment.

Another field of application of the device is to limit the detachment of jet lag by considerably shortening the times of adaptation to the new time zone.

Indeed, the device of the invention (1) is as well addressed to all travellers who frequently travel from east to west and vice versa by plane, passively undergo transitions to different time zones causing the famous jet lag or dysrhythmia or phase shift of the circadian clock. By using the device (1) for 5/10 minutes before landing, the jet lag effect reduces and the normal activities can be continued without having continuous and repeated yawns, a sense of fatigue and persistent weakness.

The term frame (2) as well means support or visor, or wearable visor, or frame for glasses internally solid.

The term frame (2) designed to cover the part of the face wherein the eyeballs are located means that said frame is suitable for covering the face area wherein the eyeballs are located.

The frame (2) can be made of different materials, for example, plastic, bioplastic, metal, aluminium, etc.

The frame (2) can comprised one or more layers, i.e. to consist of multilayer. Preferably, the frame (2) is made of at least two layers of different material. In particular, the frame (2) comprises at least one layer of material that cannot be crossed by light or of material that is impenetrable to light.

According to an embodiment, the frame (2) can consist of one or more layers of material, wherein said layer/s of material is suitable for covering or covering the part of the face where the eyeballs are located.

Preferably, the frame (2) comprises at least one layer of material that cannot be crossed by light or of material that is impenetrable to light, wherein said layer/s of material is suitable for covering or covering the part of the face where the eyeballs are located.

In the device (1) the light sources (3, 4) each have a surface area comprised from 0.15 mm² to 1 cm², or, preferably, from 0.5 mm² to 5 mm², or, more preferably, from 0.5 mm² to 2 mm².

According to a preferred embodiment, the device (1) comprises only the two light sources (3, 4), excluding the presence of other light sources.

According to a preferred embodiment, the device (1) comprises only the two light sources (3, 4), which each have a surface area comprised from 0.5 mm² to 5 mm², or, more preferably, from 0.5 mm² to 2 mm², excluding the presence of other light sources.

In the device (1) the light sources (3, 4) each have a surface area in the shape of a circumference with a diameter comprised from 2 to 2.5 mm.

In the device (1) the light sources (3, 4) each have a thickness comprised from 0.1 mm to 4 mm, or from 0.5 mm to 2.5 mm, or from 2 mm to 2.5 mm.

In the device (1) the light sources (3, 4) can each have a hemispherical shape.

In the device (1) the light sources (3, 4) can be LEDs or OLEDs or Micro-LEDs, etc.

In the case of the light sources (3, 4) consists of Micro-LEDs, the thickness of said light sources can be comprised from 0.1 and 0.3 mm.

In the device (1) the frame (2) comprises at least one layer of material suitable for preventing the passage of light from the surrounding environment.

In the device (1) the frame (2) comprises, moreover, a microprocessor or microchip (5), a battery (6), and tracks suitable for allowing the passage of electric current (7).

In the device (1) the frame (2) comprises, moreover, a microprocessor or microchip (5), a battery (6), and tracks suitable for allowing the passage of electric current (7), wherein said components are part of an integrated circuit to the device (1).

According to a preferred embodiment, in the integrated circuit the horizontal development of the electrical and electronic path has a size of 2 mm².

Preferably, the tracks suitable for allowing the passage of the electric current (7) and the microprocessor or microchip (5) are not visible to the naked eye, because they are opportunely masked or included between two layers of material forming part of the frame (2).

Preferably, only the battery (6) remains visible to allow easy access for replacement.

Therefore, according to a preferred embodiment, the microprocessor or microchip (5) and the tracks suitable for allowing the passage of electric current (7) are arranged inside the frame (2).

According to a preferred embodiment, the battery (6) is arranged on the internal surface of the frame (2). This expedient allows to easily change the battery when it runs out.

The term length means a measurement performed horizontally, with reference to the orientation of the device (1) as shown in Fig. 1.

With reference to Fig. 1, the measurement of the length of the distance between the two light emission sources (3, 4) performed horizontally and the two light emission sources (3, 4) are distant from each other by a length comprised from 55.00 to 65.00 mm, preferably from 60.00 to 65.00 mm.

With reference to Fig. 1, the distance between the two light emission sources (3, 4) is comprised from 55.00 to 65.00 mm, preferably from 60.00 to 65.00 mm.

In the device (1) the microprocessor or microchip (5) and the battery (6) are each arranged at a distance comprised between 0.5 and 5 cm with respect to any of the two distinct light emission sources (3, 4).

The device (1) has a shape and/or appearance similar to those of a traditional pair of glasses, with the difference that instead of the lenses there is part of the frame. The frame (2) is "completely solid" of material constituting the external contour of the frame.

The device (1) has a shape and/or appearance as those of a pair of glasses with a frame (2) for glasses, and wherein the internal shape of the lenses is made of the same material with which the frame (2) is made. Therefore, although the device (1) having the shape and/or appearance of a pair of glasses, it does not have the classic lenses of the glasses, replaced by the same material with which the frame is made. Moreover, at least one layer with which the frame is made, it is not penetrable or passable from the light.

According to a preferred embodiment, in the central part of the frame (2) and coinciding with the position of the eyeballs, on the innermost layer of the frame (2), there are two holes where the light sources (3, 4) are placed.

The device (1) can further comprise two temples or tape or an elastic band. The two temples or the tape or elastic band allow the device (1) to remain worn by the individual even when this assumes a vertical position, in order to prevent that the device (1) falls due to the force of gravity. Furthermore, especially the elastic tape, allows the best adherence of the device (1) to the face of the individual wearing it.

The two temples can be made of the same material with which the frame is made (2) or with different material.

The tape or elastic band is made of elastic material, preferably comprising biodegradable polymers or elastomers.

The perimeter of the internal surface part of the frame (2) is preferably covered with a layer of foam rubber with a width comprised from 5.00 to 10.00 mm, preferably the entire perimeter of the internal part of the frame (2).

Therefore, preferably, a single and continuous edge of natural foam rubber is placed inside the frame (2) in the circumference of the frame (2). Said expedient improves the darkness in the space of the eyeballs and, as a second function, it provides a comfortable and easy fit without compromising or creating any contact points between the skin of the perimeter of the eye sockets and the frame (2).

In the device (1) the two light emission sources (3, 4) are suitable for emitting light in a wavelength range comprised from 430 nm to 570 nm.

According to a preferred embodiment, in the device (1) the two light emission sources (3, 4) are suitable for emitting light in a wavelength range comprised from 450 nm to 470 nm, or to 460 nm.

According to a preferred embodiment, in the device (1) the two light emission sources (3, 4) are suitable for emitting light only in a wavelength range comprised from 430 nm to 570 nm.

According to a preferred embodiment, in the device (1) the two light emission sources (3, 4) are suitable for emitting light only in a wavelength range comprised from 450 nm to 470 nm, or only at 460 nm.

The term only means that the two light emission sources (3, 4) emit light just in the indicated wavelength range, excluding all other wavelengths.

According to a preferred embodiment, in the device (1) the two light emission sources (3, 4) are suitable for emitting intermittent light.

According to a preferred embodiment, in the device (1) the two light emission sources (3, 4) are suitable for emitting intermittent light, wherein the intermittence is achieved by means of the following sequence: 30 seconds of darkness followed by 3 continuous flashes for the duration of 5 milliseconds flash on, 5 milliseconds flash off. Sequentially, for 3 consecutive light intervals returning to the original state of 30 seconds of darkness and subsequent intervals of 5 millisecond between each flash at constant repeat.

According to a preferred embodiment, in the device (1) the two light emission sources (3, 4) are suitable for emitting intermittent light in a wavelength range comprised from 430 nm to 770 nm, preferably from 450 nm to 470 nm, or 460 nm. When the wavelength is 460 nm, the emitted light is intermittent blue light.

According to a more preferred embodiment, in the device (1) the two light emission sources (3, 4) are suitable for emitting intermittent light only in a wavelength range comprised from 430 nm to 770 nm, preferably only from 450 nm at 470 nm, or only at 460 nm. Preferably, the light sources (3, 4) consist of blue LEDs.

According to a more preferred embodiment, in the device (1) the two light emission sources (3, 4) are suitable for emitting intermittent light in a wavelength range comprised from 450 nm to 470 nm, or 460 nm, wherein the intermittence is achieved by means of the following sequence: 30 seconds of darkness followed by 3 continuous flashes for the duration of 5 milliseconds at intervals of darkness of 5 milliseconds between each flash.

According to a preferred embodiment, the device (1) indeed uses the intermittent light generated by blue lights with a frequency of 460 nm with intermittent flashes particularly effective to realign the circadian clock and/or natural biological clock. Preferably, the blue lights are light sources (3, 4) consisting of blue LEDs.

Another object is a method for manufacturing the device (1), comprised any of the embodiments above reported, comprising or, alternatively, consisting of the following steps:
A) providing a first layer of a frame (2),
B) applying on said first layer of a frame (2), a microprocessor (5), a battery (6), tracks suitable for allowing the passage of the electric current (7),
C) covering the layer obtained in step B) with a second layer of material.

Another object is a method for relaxation comprising or, alternatively, consisting of the following steps:
a) wearing a device (1) above described, by an individual;
b) operating the device so that it emits intermittent blue light.
c) keeping the intermittent blue light emission for at least 10 minutes.

According to a preferred embodiment, in step c) the blue light has a wavelength of 460 nm.

According to a preferred embodiment, in step c) the intermittence is achieved by means of the following sequence: 30 seconds of darkness followed by 3 continuous flashes for the duration of 5 milliseconds at intervals of 5 milliseconds of darkness between each flash.

According to a preferred embodiment, in step c) the blue light has a wavelength of 460 nm and the intermittence is achieved by means of the following sequence: 30 seconds of darkness followed by 3 continuous flashes for the duration of 5 milliseconds at intervals of 5 milliseconds between each flash.

Another object is the use of the device (1) as above described, including any preferred embodiment, to facilitate the post-operative recovery of surgical interventions under deep anesthesia or for relaxation of individuals.

According to a preferred embodiment, the use of the device (1) with eyes closed, preferably in a supine position or seated with the head resting on an inclined seatback in relaxation, it is much preferred.

The device (1) of the invention can be used to facilitate the post-operative recovery of surgical interventions under deep anesthesia. According to an embodiment of said use, the device (1) must be worn by the patient for at least 10 minutes, up to 6, 8 hours or until the end of the surgery. According to a preferred embodiment of said use, the light sources (3, 4) emit intermittent blue light at a wavelength of 460 nm. According to a preferred embodiment, the intermittence is achieved by means of the following sequence: 30 seconds of darkness followed by 3 continuous flashes of the duration of 5 milliseconds at intervals of 5 milliseconds of darkness between each flash.

From the above description, the advantages of the device (1) of the present invention are evident, which, although in its relatively simple embodiment, at he same time, solves many problems, with one and only one device (1).

## Claims

1. Device (1) for relaxation and/or for surgical operations under deep anesthesia and/or for the treatment of depression and/or for dysrhythmia and/or for the treatment of jet lag disorders, comprising:
- a frame (2) suitable for covering the part of the face where the eyeballs are located,
- two distinct light emission sources (3, 4) arranged on the internal surface of the frame and in front of the eyeballs suitable for emitting light,
wherein the light sources (3, 4) each have a surface area comprised from 0.15 mm² to 1 cm², or from 0.5 mm² to 5 mm², or from 0.5 mm² to 2 mm².

2. Device (1) according to claim 1, wherein the distance between the two light emission sources (3, 4) is comprised between 55.00 to 65.00 mm.

3. Device (1) according to any one of claims from 1 to 2, wherein the light sources (3, 4) each have a surface area in the shape of a circumference with a diameter comprised from 2 to 2.5 mm.

4. Device (1) according to any one of claims from 1 to 3, wherein the light sources (3, 4) each have a thickness comprising from 0.1 mm to 4 mm, or from 0.5 mm to 2.5 mm, or from 2 mm to 2.5 mm.

5. Device (1) according to any one of claims from 1 to 4, wherein the frame (2) comprises at least one layer of material that cannot be crossed by light.

6. Device (1) according to any one of claims from 1 to 5, wherein the two light emission sources (3, 4) are suitable for emitting intermittent light in a wavelength range comprised from 430 nm to 570 nm, or from 450 nm to 470 nm, or to 460 nm.

7. Device (1) according to any one of claims from 1 to 6, wherein the two light emission sources (3, 4) are suitable for emitting intermittent light, wherein the intermittence is achieved by means of the following sequence: 30 seconds of darkness followed by 3 continuous flashes for the duration of 5 milliseconds at intervals of 5 milliseconds between each flash.

8. Method for manufacturing the device (1), according to any one of the claims from 1 to 7, comprising or, alternatively, consisting of the following steps:
A) providing a first layer of a frame (2),
B) applying on said first layer of a frame (2), a microprocessor (5), a battery (6), tracks suitable for allowing the passage of electric current (7),
C) covering the layer obtained in step B) with a second layer of material.

9. Method for relaxation comprising or, alternatively, consisting of the following steps:
a) wearing a device (1), according to any one of claims 1 to 7, by an individual;
b) operating the device so that it emits intermittent blue light.
c) keeping the intermittent blue light emission for at least 10 minutes.

10. Use of the device (1) according to any one of claims from 1 to 7 to facilitate the post-operative recovery of surgical interventions under deep anesthesia or for relaxation of individuals.
